# EUROPEAN PATENT APPLICATION

(11) **EP 3 799 063 A1**
(43) Date of publication of application: **31.03.2021**
(21) Application number: 19199896.2
(22) Date of filing: 26.09.2019
(51) Int. Cl.: G16H 30/40, G16H 50/20, G01R 33/28, A61B 34/20

(54) **MRI IMAGING WITH INTERLEAVED WHITE MARKER CONTRAST PROJECTIONS FOR AI-BASED PASSIVE DEVICE TRACKING**

(71) Applicant: Siemens Healthcare GmbH, 91052 Erlangen (DE)
(72) Inventor: Maier, Florian, 91054 Buckenhof (DE); Weine, Jonathan, 44789 Bochum (DE)

(57) **Abstract**

A method for localization of an interventional device introduced into an examination object in an examination volume of a MRI system is provided. A least one white marker contrast projection of the examination volume is acquired based on at least one projection parameter. Positional information of the interventional device in the examination volume is determined using the projection. A MRI image of a slice of the examination volume including at least part of the interventional device is acquired using the determined positional and/or orientational information of the interventional device. The at least one projection parameter is adjusted, wherein adjusting the at least one projection parameter comprises applying trained functions to the at least one acquired projection, wherein the trained functions were trained with training projections associated with known positional information of an interventional device in each training projection.

## Description

Various examples of the invention generally relate to a MRI imaging method for device tracking. Various examples specifically relate to a MRI imaging method for computer-implemented passive device tracking using machine-learned (ML) algorithms. Furthermore, a corresponding MRI system and a computer program are provided.

### Background

Medical interventions are often carried out minimally invasively, wherein such techniques include for example needle biopsies, or catheter examinations. In order to carry out interventional procedures safely and efficiently, the interventional radiologist should know the exact position of the instruments used at all times. Positioning of the devices used is very important, because, for example, an inaccurate removal of tissue during a biopsy can lead to an incorrect diagnosis, or the position of the catheter is important for correct embolization.

The use of a MRI system is advantageous for monitoring and guiding instrument placement during a medical intervention due to its excellent soft tissue contrast and the absence of ionizing radiation. Online monitoring using MRI is typically performed with fast 2D MRI imaging methods. The image artifacts generated by the instruments are only visible directly in the images if the plane is optimally positioned. Due to the degrees of freedom in instrument guidance and patient movement, the instruments may no longer lie completely in the acquired plane without this being clearly recognizable from the image. Manual tracking of the 2D layers is time-consuming. 3D MRI imaging avoids the positioning problem, but yields a much lower time resolution.

### Summary

Therefore, a need exists for improved MRI techniques for device tracking that overcome or mitigate at least some of the limitations and disadvantages mentioned.

This task is solved by the features of the independent claims. Further advantageous examples of the invention are described in the dependent claims.

In the following, the solution according to the invention is described with regard to the claimed methods as well as with regard to the claimed MRI systems, wherein features, advantages, or alternative embodiments can be assigned to the other claimed objects and vice versa. In other words, the claims for the MRI systems can be improved by features described or claimed in the context of the methods. In this regard, the functional features of the methods can be performed by respective operative units of the MRI system.

A method for localization of an interventional device introduced into an examination object in an examination volume of a MRI system comprises the following steps.

In a step, at least one projection of the examination volume, or at least part of the examination volume, is acquired, wherein MR signals of the examination object are localized along a first direction, or first axis, without localizing the MR signals in a second direction, or second axis, based on at least one projection parameter.

The projection may be acquired using an off-resonance spin excitation pulse, in particular an off resonance spin excitation pulse, which excites spins in the immediate vicinity of the interventional device based on local B0 magnetic field distortions by the interventional device, which is known in the art as White Marker Contrast technique.

Therein, the device is not imaged directly as it itself does not provide a signal without water. The signal to locate the device comes from the immediate vicinity. In conventional MR images (without white marker contrast method), the image simply shows an extinguishing artifact. Not only does the device itself not deliver a signal, rather the local field distortion (without the white-marker method) also destroys signals in the immediate vicinity.

According to various examples, a one-dimensional (1D) projection is acquired, wherein the MR signals are projected onto one dimension, or axis.

In other words, in a one-dimensional (1D) projection, the MR signals from the examination volume, or at least part of the examination volume, of the MRI system may be localized along one single axis, i.e. direction or dimension, without localizing the MR signals in a second axis, or dimension, or direction. Therein, spatial positions of voxels may be only determined in relation to a single axis. For example, 1D projections may be acquired, as known in the art, by applying a single readout gradient, however other techniques known in the art may be used. The projection axis may be a single direction in 3D space, wherein any combination of the hardware axes of the MRI system may be used, i.e. any combination of at least 2 or 3 of the linear gradients along the hardware axes, may be used for generating the single gradient along the single projection axis. In other words, the MR signals may be projected onto, or on, a single dimension, or may be projected along a single direction. A projection, or 1D projection, may be acquired within one TR interval. Therefore, the method is much faster than conventional imaging.

The at least one projection parameter may be used to control the acquiring of at least one projection. For example, the at least one projection parameter may comprise one or more of an orientation of projection axis of the projection, a pulse length of a spin excitation pulse, an off-resonance shift of a spin excitation pulse, a bandwidth of a spin excitation pulse, and a receiver bandwidth of the MRI system, as will be describe in more detail in the following.

In various examples, an interventional device may be a medical instrument or device used in a medical intervention, wherein the interventional device may be made of an anorganic material, such as titanium, or another material as commonly used in the art. In various examples, the interventional device may not comprise spins, which can be excited by the MRI system, however the interventional device may induce or have localized magnetic fields around the interventional device, which may distort or shift the MRI magnetic fields in direct vicinity of the interventional device, which may be used for MR imaging of the interventional device in the MRI system.

The steps of receiving the at least one projection parameter and acquiring at least one projection may be referred to a projection sequence of the MRI system.

In another step, positional information of the interventional device in the examination volume is determined using the projection. In other words, using MR projection data, positional information of the interventional device with reference to a 3D position in world space within the examination volume of the MRI system, may be determined based thereon. The positional information may, in some embodiments, include orientational information of the interventional device.

A projection onto a single axis (1D projection) may be referred to a MR data set, comprising MR signals, which are localized along a single axis, or direction, wherein this MRI projection data set may be quickly acquired and then may be used to determine positional information of the interventional device based thereon.

In another step, an MRI image of a slice of the examination volume including at least part of the interventional device is acquired using the determined positional information of the interventional device.

In various examples, a MRI image may be a 2-dimensional MRI image of the examination object and/or of the interventional device in direct contact with the examination object, for example a 2D MRI image of a slice or disc-shaped volume of the examination volume. It will be appreciated by a person in the art that any known MRI imaging method may be used to acquire an MRI image from at least a part of the examination volume comprising at least part of the interventional device using the positional information of the interventional device.

The slice, or in other words MRI image acquisition volume, may be selected and located based on the determined positional information of the interventional device such that it includes at least part of the examination object and at least part of the interventional device. It may also be possible that the selected slice comprises only a small part of the interventional device or, in exceptions no part of the interventional device.

Acquiring the MRI image of the examination object and of the interventional device may comprise displaying, or visualizing, the MRI image to a person performing a medical intervention using the interventional device, and may be referred to as MRI visualization sequence.

In another step, the at least one projection parameter may be adjusted, wherein adjusting the at least one projection parameter comprises applying trained functions to the at least one acquired projection, wherein the trained functions were trained with training projections, respective projection parameters of each training projection, and respective known positional information of an interventional device of each training projection.

Trained functions may comprise a machine-learned algorithm, or machine-learned model, which may be applied to the at least one acquired projection and/or the at least one projection parameter. For example, as known in the art, trained functions may comprise a plurality of parameters defining how the trained functions are applied to a projection and projection parameter, and how the projection parameter is changed based on the projection. Therein, based on applying the trained functions to the MR projection, the at least one projection parameter may be adjusted, in other words corrected or changed, such that the at least one adjusted projection parameter may be used to acquire an improved MR projection of interventional device based thereon. For example, the at least one adjusted projection parameter may be used to acquire a MR projection, from which positional information of the interventional device may be determined more precisely.

In various examples, based on applying the trained functions to the at least one MR projection, positional information of the interventional device may be determined.

In an optional first step, at least one projection parameter may be received.

In another optional step, the acquired MRI image depicting the examination object and/or the interventional device, and/or the at least one adjusted projection parameter may be provided.

A method for providing trained functions for localization of an interventional device is provided in the following as a separate method, which may be performed independently of the method for determining a location of a device using the trained functions.

A method for providing trained functions for localization of an interventional device introduced into an examination object in an examination volume of a MRI system comprises the following steps.

It will be understood that training of the functions, which may be trainable functions, in order to provide trained functions, can be carried out in a plurality of iterations using a plurality of training data sets. Training the trainable functions may comprise adjusting a plurality of parameters with in the trainable functions, based on at least some of the steps as will be described in the following.

For example, a training data set may comprise a training projection of an examination object and an interventional device introduced into the examination object in an examination volume of an MRI system. The training data set may further comprise at least one projection parameter, which was used, or based on which, the training projection is acquired. The training data set may further comprise known positional information of an interventional device in the examination volume, when the training projection was acquired. The above training data may be associated with each other in order to form a training data set.

The training data set may be used to adjust at least one training projection parameter in order to acquire another projection, from which the positional data can be determined in a more precise manner, while the examination object and the interventional device are at the known position, i.e. still are defined by the known positional information. In this regard, the known positional information may be regarded as ground truth.

For example, known positional information may be known from a physical marker attached to an interventional device, or the complete training datasets may be generated by a software simulation system, simulating a MRI system with an interventional device introduced into an examination object in an examination volume of the MRI system.

In a step, a training projection of the examination volume is received, which is acquired based on at least one training projection parameter. The training projection is associated with known positional information of an interventional device in the examination volume, defining the position of the interventional device in the examination volume.

In another step, the at least one training projection parameter is adjusted, wherein adjusting the at least one training projection parameter comprises applying functions, or trainable functions, to the training projection and/or the at least one training projection parameter.

In another step, a projection of the examination volume is acquired based on the at least one adjusted training parameter.

In another step, positional information of the interventional device in the examination volume is determined from the acquired projection.

In another step, a difference between the determined positional information and the known positional information (as ground truth) is determined for the interventional device.

For example, a difference between the determined positional information and the known positional information may be a difference between an orientation between a main axis, i.e. a longitudinal axis, of the interventional device, and the orientation of the projection axis.

In addition, or instead of the difference between the determined positional information and the known positional information, an uncertainty of the determined positional information may be determined statistically.

The statistically determinable uncertainty of the determined positional information may be used to optimize the projections. Accordingly, the next projection may be selected in such a way that the overall uncertainty is reduced, or minimized.

In a further step, the functions are trained based on the determined difference. Training the functions may comprise adjusting the plurality of parameters in the trainable functions, such that the determined difference is minimized. The functions may be further trained on the the statistically determined uncertainty of the determined positional information, specifically in such a way, that the uncertainty is minimized.

The invention is based on the concept that MR data acquisition for instrument localization may be carried out in an alternating or interleaved manner, with MRI imaging for visualization of an examination object for an interventional radiologist, wherein the instrument localization may be performed and controlled by a computer-implemented method, in particular, based on machine-learning (ML) techniques. The localization data may be used to position both the MRI imaging volume, in order to visualize the examination object and the interventional device to a user of the MRI system, and to improve the MR data acquisition for localization of the interventional device in a following iteration of the method.

Thereby, the method according to the present disclosure enables computer-controlled instrument localization by an interleaved sequence of steps according to an AI-controlled projection method for instrument localization and a known MRI imaging method.

Advantages of the techniques according to the invention may be a shortening of a medical intervention, since manual steps for instrument tracking are omitted, a saving of a human assistant at the host system by increased robustness of the computer-implemented method according to the invention, as well as an increase of the position accuracy and position reproducibility. Furthermore, a better treatment is possible by a more exact positioning. The spatially non-selective excitation enables robust detection of the instrument. Properly positioned imaging planes are not a prerequisite. The AI-based tracking for estimating the instrument position allows a situation-specific optimized imaging for tracking purposes. This increases robustness and allows accelerated imaging. In particular, the estimation of the instrument position from the projection data with situation-optimized acquisition of the projections permits more robust instrument tracking. This shortens the intervention and saves assistant time.

A MRI system is configured to carry out a method for localization of an interventional device in a MRI system according to the invention. The MRI system comprises a MRI control unit and a memory unit, wherein the memory unit stores control information executable by the MRI control unit, and wherein the MRI system is configured to perform the following steps when executing the control information in the MRI control unit.

In a first step, at least one projection of the examination volume is acquired, in which MR signals of the examination object are projected onto a first projection axis without localizing the MR signals in a second direction perpendicular to the first direction, based on at least one projection parameter.

In another step, positional information of the interventional device in the examination volume is determined using the projection.

In another step, a MRI image of a slice of the examination volume including at least part of the interventional device is acquired using the determined positional information of the interventional device.

In another step, the at least one projection parameter is adjusted, wherein adjusting the at least one projection parameter comprises applying trained functions to the at least one acquired projection, wherein the trained functions were trained with training projections, respective projection parameters of each training projection, and respective known positional information of an interventional device of each training projection.

The MRI equipment may be further configured to perform any method or combination of methods according to the present disclosure.

A computer program, or computer program product, which is directly loadable into a memory of a MRI controller of a MRI system, comprises instructions which, when the program is executed by a controller of a MRI system, cause the MRI system to execute the steps of any method according to the present disclosure.

An electronically readable data carrier comprises electronically readable control information stored thereon, which is configured such that, when executed in a MRI control unit of a MRI system, it causes the MRI system to execute the steps of any method according to the present disclosure.

For such a MRI system, a computer program and an electronically readable data carrier, technical effects can be achieved which correspond to the technical effects for the methods according to the present disclosure.

Although the specific features described in the above summary and the following detailed description are described in relation to specific examples, it is understood that the features may not only be used in the respective combinations, but may also be used in isolation or in any combination, and features from different examples of methods, MRI systems, computer programs and electronically readable media may be combined and correlated with each other, unless expressly stated otherwise.

It should therefore be understood that a method according to the present disclosure can be improved with any feature described in connection with a MRI system according to the present disclosure and that a MRI system can be improved with any feature described in connection with any of the methods. In particular, a MRI system may be configured so that steps of the machine learning methods are performed by a computing device of the MRI system.

It is to be appreciated, that the solution according to the invention is described with respect to methods and systems for determining a location of a device by applying trained functions as well as with respect to methods and systems for providing trained functions for determining a location of a device. Features, advantages, or alternative embodiments herein may be assigned to the other claimed objects and vice versa. In other words, claims for methods and systems for providing trained functions for determining a location of a device may be improved with features described or claimed in context of the methods and systems by applying trained functions, and vice versa. Specifically, in the claims related to applying trained functions, the trained functions may be improved with features described for the methods for providing trained functions. Likewise, projection parameters and images may be improved with features described for the training projection parameters and images, and vice versa.

The above summary is therefore only intended to give a brief overview of some features of some embodiments and implementations and is not to be understood as a limitation. Other embodiments may include features other than those described above.

### Short description of the drawings

The invention is described in more detail below with reference to the accompanying drawings.
- Figure 1: schematically illustrates a MRI system with which the methods for localization of an interventional device can be carried out, according to embodiments of the invention.
- Figure 2: illustrates a flowchart with steps for localization of an interventional device, according to embodiments of the invention.
- Figure 3: illustrates a flowchart with steps for providing trained functions for localization of an interventional device, according to embodiments of the invention.

### Detailed description

The above-mentioned elements, features, steps, and concepts according to the present disclosure will be explained in the following detailed description by means of exemplary embodiments, which are explained with reference to the attached drawings.

The drawings are to be regarded as schematic drawings and the elements depicted in the drawings are not necessarily shown to scale. Rather, the various elements are presented in such a way that their function and general purpose can be understood by a person skilled in the art. Any connection or coupling between functional blocks, devices, components or other physical or functional units described in the drawings or herein may also be made by indirect connection or coupling. A coupling between the components can also be established via a wireless connection. Function blocks can be implemented in hardware, firmware, software, or a combination thereof.

In the following, embodiments of the invention are described in detail with reference to the accompanying drawings. It should be noted that the following description of the execution examples is not to be understood in a narrow sense. The scope of the invention shall not be limited by the execution examples described in the following or by the drawings, which are for illustration purposes only.

Examples of the present disclosure relate to a MRI imaging method with interleaved white marker contrast projections for AI-based passive instrument tracking. In particular, a computer-implemented method is provided for computer-implemented automatic positional adjustment of 2D-visualization images for visualization of a medical interventional device, based on 1D-projections controlled by a machine-learned algorithm. In particular, a MRI imaging method is provided for instrument tracking, or needle tracking, in MR-guided percutaneous interventions or interventions, which are, in other words, MRI guidance techniques.

Many medical interventions are carried out minimally invasively. These include, for example, needle biopsies, or catheter examinations. The positioning of the devices used is very important here, because, for example, an inaccurate removal of tissue during a biopsy can lead to an incorrect diagnosis, or the position of the catheter is important for correct embolization. The use of a MRI scanner is recommended for monitoring and guiding instrument positioning due to its excellent soft tissue contrast and the absence of ionizing radiation. Online monitoring using MRI techniques is typically performed with fast 2D slice images. The image artifacts generated by the instruments are only visible directly in the MRI images if the plane, i.e. 2D layer, is optimally positioned. 3D imaging avoids the positioning problem, but results in a significantly lower time resolution. Due to the degrees of freedom in instrument guidance and patient movement, the instruments may no longer lie completely in the acquired slice without this being clearly recognizable from the image.

In order to carry out interventional procedures safely and efficiently, the interventional radiologist should know the exact position of the instruments used at all times. Manual tracking of the 2D slice is time-consuming.

Needle tracking was previously performed using Template Matching algorithms, as known from the document US 8,526,691 B2. This usually works quite well in phantoms. In patients with different tissue types (e.g. subcutaneous fat, muscle, liver, ribs and various transitions), however, the robustness of the techniques may be poor.

In the past, additional MR-visible markers were also used for slice positioning, as known from the document Maier F. et al, "3D passive marker tracking for MR-guided interventions" in Proceedings of the 19th Annual Meeting of ISMRM, 2011. However, these lead to the problem that they make the handling of the needle more difficult, for example because they reduce the penetration depth.

3D projections with white marker contrast are known from the document Patil S. et al. "Automatic slice positioning (ASP) for passive real-time tracking of interventional devices using projection-reconstruction imaging with echo-dephasing (PRIDE)" in Magnetic Resonance in Medicine: An Official Journal of the International Society for Magnetic Resonance in Medicine 62.4 (2009): 935-942.

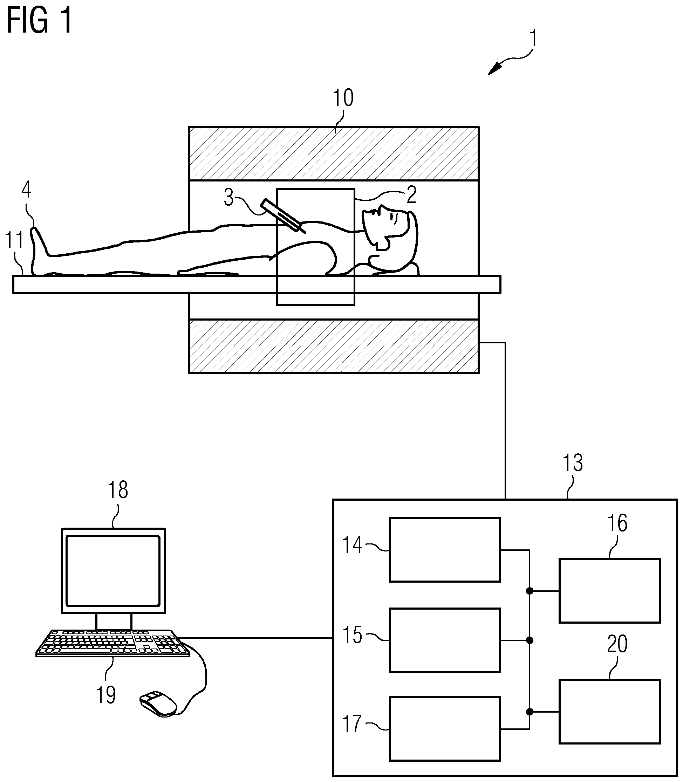

Figure 1 schematically illustrates a MRI system with which the methods for localization of an interventional device can be carried out, according to embodiments of the invention.

An examination person, or more generally an examination object 4, is positioned in the tunnel of MRI system 1. In an examination volume 2 of the MRI system 1, an interventional device 3, such as for example a surgical instrument is introduced into the examination object 4. The MRI system 1 has a magnet 10 for generating a basic magnetic field BO, whereby an examination person 4 arranged on a table 11 is driven into the center of the magnet to acquire location-coded magnetic resonance signals from an examination volume 4. By irradiating high-frequency pulse sequences and switching magnetic field gradients, the magnetization produced by the base field BO can be disturbed by deflection of the nuclear spins from their equilibrium position, and the currents induced in receiving coils when returning to their equilibrium position can be converted into magnetic resonance signals. The general functionality for the acquisition of MRI images and MRI projections and the detection of magnetic resonance signals are known to the person skilled in the art, so that a detailed explanation is omitted.

The MRI system 1 also has a MRI control unit 13, which is used to control the MRI system. The central MRI control unit 13, which is designed such that the method described below for device localization is carried out, has a gradient control 14 for controlling and switching the magnetic field gradients and an RF control 15 for controlling and irradiating the RF pulses for deflecting the nuclear spin from its equilibrium position. In a memory unit 16, for example, the parameters necessary for the acquisition of MRI images and MRI projections can be stored, as well as all programs required for the operation of MRI system 1. An acquisition unit 17 controls the image acquisition and thus controls the sequence of the magnetic field gradients and HF pulses as well as the reception intervals of MRI signals depending on the selected parameters. Thus, the acquisition unit 17 also controls the gradient control 14 and the RF control 15. MRI images can be generated in a computing unit 20, and can be displayed on a display 18, on which for example images displaying the examination object 4 and the interventional device 3 are depicted to a person performing a medical intervention, i.e. operating the interventional device 3. The display 18 may be used for online monitoring of the position of the interventional device in a medical intervention. An operator can operate MRI system 1 via an input unit 19. The memory unit 16 can include MRI measurement sequences and computer programs which, when executed in the computing unit 20 shown, perform a method according to the present disclosure. The MRI control unit 13 may also be designed to improve a device tracking technique as explained in detail below. In particular, the memory unit stores 16 control information that can be executed by the MRI control unit 13. Furthermore, the acquisition unit 17 is designed in such a way that it can perform the device tracking techniques as described below.

In general, receiving data may comprise, receiving data in a current iteration of steps of a method, which have been provided from a previous iteration of the steps of the method, or receiving stored data from an internal or external memory of a computing device or a MRI system, or receiving data from another computing device, wherein in general any method of sending and receiving data may be implemented.

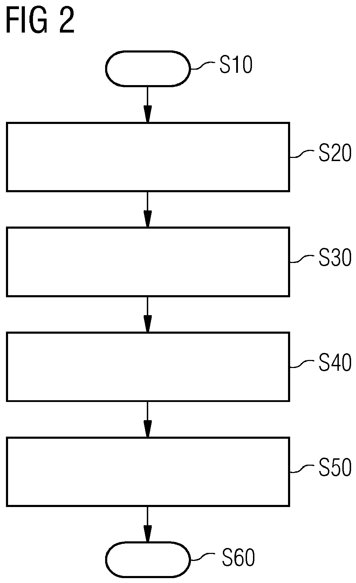

Figure 2 illustrates a flowchart with steps for localization of an interventional device introduced into an examination object in an examination volume in a MRI system, according to embodiments of the invention.

The method starts in step S10. In step S20, at least one projection of the examination volume is acquired, in which MR signals of the examination object are projected onto a first direction without localizing the MR signals in a second direction perpendicular to the first direction, based on at least one projection parameter.

In step S30, positional information of the interventional device in the examination volume is determined using the projection.

In step S40, a MRI image of a slice of the examination volume including at least part of the interventional device is acquired using the determined positional information of the interventional device. Therein, the slice may be positioned based on the positional information of the interventional device, such that the interventional device, or distortions based on the interventional device, are clearly visible in the MRI visualization images.

In step S50, the at least one projection parameter is adjusted, wherein adjusting the at least one projection parameter comprises applying trained functions to the at least one acquired projection, wherein the trained functions were trained with training projections, respective projection parameters of each training projection, and respective known positional information of an interventional device of each training projection. The method ends in step S60.

Steps S20 to S50 may be referred to a as a iteration of the techniques according to the invention, wherein steps S20 to S50 may be repeated in various continuous iterations, in order to provide continuously optimized visualization by the MRI system, e.g. for a person performing a medical intervention using the interventional device.

Various examples of the present disclosure will be described in the following in more detail, wherein instrument localization scans are carried out alternately or interleaved with 2D layer scans, in order to provide clear visualization based on an optimal MRI imaging slice position for an interventional radiologist. Interleaving of steps of the method may be performed at the repetition level so that the instrument position can be checked during MRI image acquisition.

The following logical blocks are interlaced with each other:
1. Projection block
2. 2D-layer image and optionally artefact segmentation

Blocks 1 and 2 may be repeated iteratively. In block 2, a complete 2D image or part of an image may be acquired, however as a smallest part of a 2D image, in block 2, a single repetition step (TR), or a plurality of repetition steps (TR) may be performed.

In the projection block, off-resonant excitation and readout are carried out, so that mainly the direct instrument environment provides the signal. In such a way, a positive contrast is generated, which only represents the strong B0 field distortion in the instrument environment (also referred to as White Marker Contrast). The excitation pulse can be played out with a long pulse duration to achieve narrowband excitation. The excitation takes place without selection gradients and is therefore not spatially selective. 1D projections are read out.

The orientation of the projection axis may vary and may be selected so that the instrument position can be determined as accurately as possible, i.e. mostly in such a way, that critical (characteristic) parts or the biggest portion of the interventional device lie within the imaging volume. At the beginning, at least three projections are necessary to precisely determine the position of the device in three-dimensional space. Once the instrument position is known to the AI-algorithm, position tracking can be performed with fewer projections if necessary, since the movement of the instrument in the patient's body typically has fewer degrees of freedom, and the shape or form of the instrument is known.

The optimal projection axis of the projections can be formulated as part of an optimization problem. The used AI algorithm is based on reinforcement learning, where training projections serve as input and projection parameters, such as for example orientations of projection axes, and/or the off-resonant excitation pulse duration, and/or the off-resonance shift, and/or the receiver bandwidth define the state of the MRI system (MRI scanner). The reward function during the training process is determined by how similar the orientation (direction) of the projection is to the orientation of the needle. The orientation of the needle must be known. This can be ensured, for example, by external position determination or by simulated data. By this formulation of the problem, it is possible to use feedback of the surgeon at the end of an intervention for the continuous improvement of the slice positioning.

The projection block used can be spin echo based, as the intra-voxel phasing around the instrument can be better compensated.

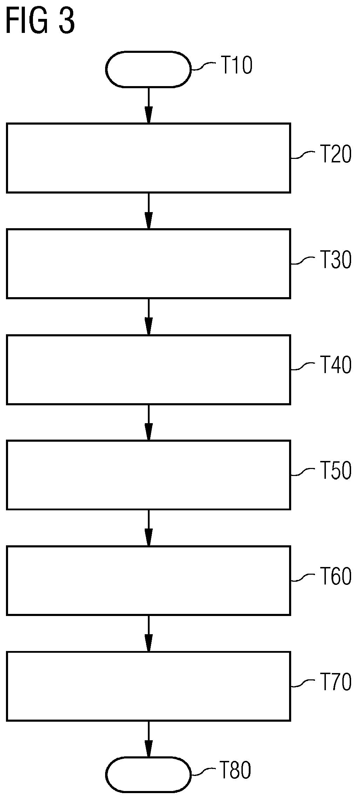

Figure 3 illustrates a flowchart with steps for providing trained functions for localization of an interventional device introduced into an examination object in an examination volume in a MRI system, according to embodiments of the invention.

The method starts in step T10. In step T20, a training projection of the examination volume is received, which is acquired based on at least one training projection parameter. The training projection is associated with known positional information of an interventional device in the examination volume, defining the position of the interventional device in the examination volume.

In step T30, the at least one training projection parameter is adjusted, wherein adjusting the at least one training projection parameter comprises applying functions, or trainable functions, to the training projection and/or the at least one training projection parameter.

In step T40, a projection of the examination volume is acquired based on the at least one adjusted training parameter.

In step T50, positional information of the interventional device in the examination volume is determined from the acquired projection.

In step T60, a difference between the determined positional information and the known positional information (as ground truth) is determined for the interventional device.

For example, a difference between the determined positional information and the known positional information may be a difference between an orientation between a main axis, i.e. a longitudinal axis, of the interventional device, and the projection axis.

In step T70, the functions are trained based on the determined difference. Training the functions may comprise adjusting the plurality of parameters of the trainable functions, such that the determined difference becomes small. The method ends in step T80.

In general, examples of the present disclosure include a variety of circuits, memories, interfaces, or electrical processing devices such as processors in an MRI system. All references to these units and other electrical devices and the functions they provided are not limited to what is illustrated and described. While certain designations may be assigned to different circuits or other disclosed electrical devices, these designations are not intended to restrict the functionality of the circuits and other electrical devices. These circuits and other electrical equipment may be combined and/or separated according to the electrical design. It is to be understood that any disclosed circuit or other electrical device may include any number of microcontrollers, graphics processing units (GPU), integrated circuits, memory devices such as FLASH, random access memory (RAM), read-only memory (ROM), electrically programmable read-only memory (EPROM), electrically erasable programmable read-only memory (EEPROM), or any other suitable embodiment thereof, and software, which cooperate with each other to perform the methods disclosed herein. In addition, each of the electrical devices may be configured to execute program code contained in a computer-readable medium and configured to execute any number of steps in accordance with the methods according to the present disclosure.

From the above said, some general conclusions may be drawn:
Various techniques according to the present disclosure may provide for passive device tracking, wherein no dedicated hardware, in particular no dedicated tracking coils in a MRI system or markers on interventional devices, are used in the techniques according to the invention.

It may be possible that determining positional information of the interventional device in the examining volume comprises applying the trained functions to the at least one acquired projection, wherein the trained functions were trained with training projections, respective projection parameters of each training projection, and respective known positional information of an interventional device of each training projection. In other words, the determining of positional information from the projection and the adjusting of the at least one projection parameter may be performed in one method step comprising applying the trained functions to the MRI projection. Similarly, in the method for providing trained functions, the step of determining positional information and adjusting the training projection parameters may be performed in a single step comprising applying the trained functions to the training projection.

Acquiring at least one projection of the examination volume may comprise exciting spins in the examination volume by an off-resonance spin excitation pulse, wherein the off-resonance spin excitation pulse has a bandwidth, which does not comprise a bandwidth of a spin excitation pulse used for acquiring the MRI image. In other words, for acquiring a projection, an off-resonant HF spin excitation pulse may be used, which may be spaced from the HF excitation pulse of the MRI imaging sequence, and/or which may not comprise the water resonant frequency of the MRI imaging sequence.

The positional information may comprise one or more of a location of the interventional device in the examination volume of the MRI system, an orientation of the interventional device in the examination volume of the MRI system, and a dimension, or size of the interventional device.

The steps of the method may be carried out in each of a plurality of iterations, wherein, in a directly following iteration, the acquiring of at least one projection of the examination volume may be based on the at least one adjusted projection parameter of the directly previous iteration.

In other words, in a current iteration or repetition of the steps according to the invention, at least one projection parameter may be received, which may be the at least one adjusted projection parameter of a previous, specifically directly preceding, iteration.

The iterations may be performed in a continuous process, such as to visualize the interventional device in relation to, or together with, the examination object to a user of the MRI performing a medical intervention using the device on the examination object.

In a first iteration, the positional information of the interventional device may not be precise enough, so that in the first iteration the at least one projection may comprise three projections in different directions, in some examples at least 6 different directions, in order to determine a position and orientation of an interventional device in 3D.

Further, in each of following iterations, the at least one projection comprises less than three projections, specifically only one projection onto a single axis, in order to continuously improve and adjust the at least one projection parameter, to follow the interventional device.

The techniques according to the present disclosure are based on the finding that and interventional device is moved by an operator using the interventional device and a medical intervention, wherein if the exact position and form or shape of the interventional device is known, a faster and more rudimental MRI image acquisition, or MRI data acquisition, i.e. based on a projection only, may be used to determine the moving position of the interventional device.

The steps of the MRI visualization sequence and the MRI projection sequence may be performed in an alternating, specifically in an interleaved sequence. In particular, acquiring at least one projection of the examination volume, and acquiring a MRI image of a slice of the examination volume, may be carried out in an interleaved sequence, wherein the MRI projection acquisition may be performed in between the acquisition of different parts of k-space data of the MRI visualization sequence. For example, after acquiring a plurality of k-space lines, the MRI projection may be acquired, and after that, further k-space lines may be acquired in the MRI visualization sequence. In other words, acquiring at least one projection of the examination volume may be interleaved with acquiring a MRI image of a slice of the examination volume on a repetition level. In such a way, the projection may be acquired while visualizing the MRI image to the user of the MRI system.

Acquiring a projection may be performed using a spin-echo based technique.

Acquiring a projection may comprise exciting spins in the examination volume using a spatially non-selective spin excitation pulse.

Acquiring a projection may comprise exciting spins in the examination volume using a excitation pulse with a bandwidth optimized by the algorithm, and/or a excitation pulse with a pulse length optimized by the algorithm, and/or an off-resonant spin excitation pulse, wherein the frequency of the spin excitation pulse is offset from the water resonance frequency of the examination object. The offset may be optimized by the algorithm.

The at least one projection, or in general, any projection or training projection according to the present disclosure, may be acquired using a White Marker Contrast technique.

The at least one projection parameter may comprise one or more of an orientation of a projection axis of the projection, a pulse length of a spin excitation pulse for acquiring a projection, an off-resonance shift of a spin excitation pulse for acquiring a projection, a bandwidth of a spin excitation pulse for acquiring a projection, and a receiver bandwidth of the MRI system for acquiring a projection.

The projections may be ID-projections of the examination volume onto a single projection axis, i.e. a single projection axis along a projection direction, and the MRI images may be 2D-images of a slice of the examination volume of the MRI system.

Acquiring an MRI image for visualization of the examination object and the interventional device may comprise an artefact segmentation of the MRI image.

Summarizing, a method is provided for localization of an interventional device in a MRI system, as well as a corresponding MRI system, which enable fast and reliable online visualization of an interventional device during a medical intervention.

A medical intervention may be shortened, since manual steps for instrument tracking are omitted, wherein properly positioned slices are not a prerequisite for starting the medical intervention. In such a way, a better treatment is enabled by exact positioning of the interventional device.

Although the invention was illustrated and described with regard to certain preferred embodiments, equivalents and changes will be made by persons skilled in the art after reading and understanding the description. This invention covers all such equivalents and modifications and is limited only by the scope of the attached claims.

## Claims

1. Method for localization of an interventional device (3) introduced into an examination object (4) in an examination volume (2) of a MRI system (1), comprising:
- Acquiring at least one projection of the examination volume (2), in which MR signals of the examination object are projected onto a first projection axis without localizing the MR signals along a second direction, based on at least one projection parameter;
- Determining positional information of the interventional device (3) in the examination volume (2) using the projection; and
- Acquiring a MRI image of a slice of the examination volume (2) including at least part of the interventional device (3) using the determined positional information of the interventional device (3); and
- Adjusting the at least one projection parameter, wherein adjusting the at least one projection parameter comprises applying trained functions to the at least one acquired projection, wherein the trained functions were trained with training projections, respective training projection parameters of each training projection, and respective known positional information of an interventional device (3) of each training projection.

2. Method according to claim 1, wherein acquiring at least one projection of the examination volume (2) comprises exciting spins in the examination volume (2) by an off-resonance HF spin excitation pulse, wherein the off-resonance spin excitation pulse has a bandwidth, which does not comprise a bandwidth of a spin excitation pulse used for acquiring the MRI image.

3. Method according to claim 1 or 2, wherein the positional information comprises one or more of a location of the interventional device (3), an orientation of the interventional device (3), and a dimension of the interventional device (3).

4. Method according to one of the preceding claims, wherein the steps of the method are carried out in each of a plurality of iterations, wherein, in a directly following iteration, the acquiring of at least one projection of the examination volume (2) is based on the at least one adjusted projection parameter provided from a directly previous iteration.

5. Method according to claim 4, wherein in a first iteration the at least one projection comprises at least three projections onto three different projection axes, and wherein in each of the following iterations the at least one projection comprises only one projection onto one axis.

6. Method according to one of the preceding claims, wherein the steps of acquiring at least one projection of the examination volume (2), and acquiring a MRI image of a slice of the examination volume (2) are carried out in an interleaved sequence.

7. Method according to one of the preceding claims, further comprising:
- Receiving at least one projection parameter; and
- Providing the acquired MRI image depicting the examination object (4) and the interventional device (3) and/or the at least one adjusted projection parameter;

8. Method for providing trained functions for localisation of an interventional device (3) introduced into an examination object (4) in an examination volume (2) of a MRI system (1), comprising:
- Receiving a training projection of the examination volume (2) based on at least one training projection parameter, together with known positional and/or orientational information of the interventional device (3) in the examination volume (2);
- Adjusting the at least one training projection parameter, wherein adjusting the at least one training projection parameter comprises applying functions to the training projection;
- Acquiring a projection of the examination volume (2) based on the at least one adjusted training parameter;
- Determining positional information of the interventional device (3) in the examination volume (2) from the acquired projection;
- Determining a difference between the determined positional and/or orientational information and the known positional and/or orientational information of the interventional device (3);
- Determining uncertainty of the determined positional information and/or orientational information based on the history of already acquired projections; and
- Training the functions based on the determined difference and uncertainty.

9. Method according to one of the preceding claims, wherein acquiring a projection is performed comprising one or more of a spin-echo based technique, a spatially non-selective spin excitation pulse, a excitation pulse with a bandwidth optimized by the algorithm, and a excitation pulse with a pulse length optimized by the algorithm, an off-resonant spin excitation pulse, wherein the bandwidth of the spin excitation pulse is offset from the water resonance frequency of the examination object, wherein the offset is optimized by the algorithm.

10. Method according to one of the preceding claims, wherein the at least one projection is acquired using a White Marker Contrast technique.

11. Method according to one of the preceding claims, wherein the at least one projection parameter comprises one or more of an orientation of the projection axis, a pulse length of a spin excitation pulse, an off-resonance shift of a spin excitation pulse, a bandwidth of a spin excitation pulse, and a receiver bandwidth of the MRI system (1).

12. Method according to one of the preceding claims, wherein the projections are ID-projections of the examination volume (2) onto a projection axis, and wherein the MRI images are 2D-images of a slice of the examination volume (2) of the MRI system (1).

13. Method according to one of the preceding claims, wherein acquiring at least one projection of the examination volume (2) is interleaved with acquiring a MRI image of a slice of the examination volume (2) on repetition level.

14. MRI system (1) configured for localisation of an interventional device (3) introduced into an examination object (4) in an examination volume (2) of the MRI system (1), the MRI system (1) comprising a MRI control unit (13) and a memory unit (16), wherein the memory unit (16) stores control information executable by the MRI control unit (13), and wherein, when executing of the control information in the MRI control unit (13), the MRI system (1) is adapted to perform the following steps:
- Acquiring at least one projection of the examination volume (2), in which MR signals of the examination object are projected onto a first projection axis without localizing the MR signals along a second direction, based on at least one projection parameter;
- Determining positional information of the interventional device (3) in the examination volume (2) using the projection; and
- Acquiring a MRI image of a slice of the examination volume (2) including at least part of the interventional device (3) using the determined positional information of the interventional device (3); and
- Adjusting the at least one projection parameter, wherein adjusting the at least one projection parameter comprises applying trained functions to the at least one acquired projection, wherein the trained functions were trained with training projections, respective training projection parameters of each training projection, and respective known positional information of an interventional device (3) of each training projection.

15. Computer program, which can be loaded into a memory unit (16) of a MRI control unit (13) of a MRI system (1) and comprises instructions, which, when executed by the control unit (13) of the MRI system (1), cause the MRI system (1) to carry out the method according to one of claims 1 to 13.
